(19) Europäisches Patentamt
European Patent Office
Office européen des brevets

(11) **EP 4 491 711 A1**

## (12) EUROPEAN PATENT APPLICATION
published in accordance with Art. 153(4) EPC

(43) Date of publication:
**15.01.2025 Bulletin 2025/03**

(21) Application number: **23766878.5**

(22) Date of filing: **08.03.2023**

(51) International Patent Classification (IPC):
**C12N 5/071** (2010.01)

(52) Cooperative Patent Classification (CPC):
**C12N 5/06**

(86) International application number:
**PCT/JP2023/008765**

(87) International publication number:
**WO 2023/171702 (14.09.2023 Gazette 2023/37)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.03.2022 US 202263317552 P**

(71) Applicant: **Keio University
Tokyo, 108-8345 (JP)**

(72) Inventors:
• **SATO, Toshiro
Tokyo 160-8582 (JP)**
• **TAKANO, Ai
Tokyo 160-8582 (JP)**

(74) Representative: **Cabinet Becker et Associés
25, rue Louis le Grand
75002 Paris (FR)**

## (54) PRODUCTION METHOD FOR ORGANOID

(57) What is provided is a production method for an organoid, including a step of dissociating a biological tissue and a step of carrying out suspension culture of the dissociated biological tissue in a state of being dispersed in a medium to form an organoid, in which the medium is substantially free of a p38 inhibitor and contains the following components (i) to (iv): (i) 0.1% to 10% by volume of an extracellular matrix; (ii) insulin-like growth factor 1 (IGF-1); (iii) fibroblast growth factor 2 (FGF-2); and (iv) at least one selected from the group consisting of a Wnt agonist, a bone morphogenetic protein (BMP) inhibitor, a transforming growth factor-$\beta$ (TGF-$\beta$) inhibitor, and an epidermal growth factor (EGF).

EP 4 491 711 A1

**Description**

TECHNICAL FIELD

The present invention relates to a production method for an organoid. More specifically, the present invention relates to a production method for an organoid and a screening method for an anticancer drug. Priority is claimed on US provisional application No. US 63/317,552, filed in the United States on March 8, 2022, the content of which is incorporated herein by reference.

BACKGROUND ART

Drug screening, which comprehensively searches for the effects of a large number of therapeutic drug candidates, is a fundamental technology in drug discovery. In cancer drug discovery in the related art, a method has been adopted in which primary screening is carried out using a cancer cell line established from a patient-derived cancer tissue, and safety and side effects are confirmed using an animal model.

However, cancer cell lines may lack many of the properties of actual clinical tumors and the sensitivity to anticancer drugs. In addition, even in a case where cancer occurs in the same organ, the cancer exhibits different genetic and biological characteristics from patient to patient. It is difficult to comprehensively cover this diversity in cancer cell lines. As a result, many of the anticancer drug candidates selected using cancer cell lines have not yet been applied clinically, and this "valley of death" in drug discovery research has become a major medical and social issue.

The present inventors have pioneered the development of an organoid technology for permanent three-dimensional culture of tissue stem cells (see, for example,

Non-Patent Documents 1 and 2). The present inventors have also constructed cancer organoid biobanks for colorectal cancer, pancreatic cancer, gastric cancer, and the like by applying the organoid technology to various patient-derived cancer tissues (see, for example, Non-Patent Documents 3 to 5).

Patient-derived cancer organoids retain many of the characteristics of cancer tissue of the patient, and cancer organoid biobanks are intended to be applied as next-generation cancer research resources to basic cancer research, cancer diagnostic tools, drug discovery, personalized treatment, and the like.

Recent overseas clinical trials have shown that the drug sensitivity of patient-derived cancer organoids correlates with the response of clinical tumors to anticancer drugs, and there are also hopes for the implementation of organoid medicine, which uses organoids to predict the therapeutic effects of chemotherapy.

Citation List

Non-Patent Documents

Non-Patent Document 1: Sato T, et al., Single Lgr5 stem cells build crypt-villus structures in vitro without a mesenchymal niche, Nature, 459, 262-266, 2009.
Non-Patent Document 2: Sato T, et al., Long-term Expansion of Epithelial Organoids From Human Colon, Adenoma, Adenocarcinoma, and Barrett's Epithelium, Gastroenterology, 141, 1762-1772, 2011.
Non-Patent Document 3: Fujii M, et al., A Colorectal Tumor Organoid Library Demonstrates Progressive Loss of Niche Factor Requirements during Tumorigenesis, Cell Stem Cell, 18, 827-838, 2016.
Non-Patent Document 4: Seino T, et al., Human Pancreatic Tumor Organoids Reveal Loss of Stem Cell Niche Factor Dependence during Disease Progression, Cell Stem Cell, 22, 454-467, 2018.
Non-Patent Document 5: Nanki K, et al., Divergent Routes toward Wnt and R-spondin Niche Independency during Human Gastric Carcinogenesis, Cell, 174, 856-869, 2018.

SUMMARY OF INVENTION

Technical Problem

However, it is difficult to evaluate side effects and safety on normal tissues with drug screening in the related art. It is possible to culture a normal tissue by using the organoid technology. However, it has been difficult to efficiently carry out large-scale culture of a normal tissue-derived organoid with culture technologies in the related art. For this reason, it was not possible to introduce the normal tissue-derived organoid into drug screening.

Therefore, an object of the present invention is to provide a technology that efficiently enables large-scale culture of an organoid, regardless of whether the organoid is derived from a normal tissue or derived from a tumor tissue.

Solution to Problem

The present invention includes the following aspects.

[1] A production method for an organoid, including a step of dissociating a biological tissue and a step of carrying out suspension culture of the dissociated biological tissue in a state of being dispersed in a medium to form an organoid, in which the medium is substantially free of a p38 inhibitor and contains the following components (i) to (iv).

(i) 0.1% to 10% by volume of an extracellular matrix
(ii) insulin-like growth factor 1 (IGF-1)
(iii) fibroblast growth factor 2 (FGF-2)
(iv) at least one selected from the group consisting of a Wnt agonist, a bone morphogenetic protein (BMP) inhibitor, a transforming growth factor-$\beta$ (TGF-$\beta$) inhibitor, and an epidermal growth factor (EGF)

[2] The production method according to [1], in which the suspension culture is carried out while stirring the medium.
[3] The production method according to [1] or [2], in which the biological tissue is a normal tissue.
[4] The production method according to any one of [1] to [3], in which the Wnt agonist is at least one selected from the group consisting of a Wnt protein, an R-spondin, and a GSK-3$\beta$ inhibitor.
[5] The production method according to any one of [1] to [4], in which the Wnt agonist is a Wnt protein and an R-spondin.
[6] The production method according to [5], in which the Wnt protein is a complex with afamin.
[7] A screening method for an anticancer drug, including a step of carrying out suspension culture of a normal tissue-derived organoid and a tumor tissue-derived organoid in a state of being dispersed in a medium in a presence of a test substance; and a step of evaluating an effect of the test substance on the normal tissue-derived organoid and the tumor tissue-derived organoid, in which the test substance that has few side effects on the normal tissue-derived organoid and is effective on the tumor tissue-derived organoid is shown to be an anticancer drug that is effective against the tumor tissue, and the medium is substantially free of a p38 inhibitor and contains the following components (i) to (iv).

(i) 0.1% to 10% by volume of an extracellular matrix
(ii) IGF-1
(iii) FGF-2
(iv) at least one selected from the group consisting of a Wnt agonist, a BMP inhibitor, a TGF-$\beta$ inhibitor, and an EGF

[8] The screening method according to [7], in which the normal tissue-derived organoid is an organoid produced by the production method according to [3]. Advantageous Effects of Invention

According to the present invention, it is possible to provide a technology that efficiently enables large-scale culture of an organoid, regardless of whether the organoid is derived from a normal tissue or derived from a tumor tissue.

BRIEF DESCRIPTION OF DRAWINGS

[FIG. 1] is a graph showing the results of measuring the proliferation of an organoid in Experimental Example 1.
[FIG. 2] is a graph showing the results of calculating a doubling time of an organoid in Experimental Example 1.
[FIG. 3] is a graph showing the results of measuring the proliferation of an organoid in Experimental Example 2.
[FIG. 4] is a graph showing the results of calculating a coefficient of variation of an area of a fluorescent dye in Experimental Example 2.
[FIG. 5] is a graph showing the results of calculating a Z'-factor for each organoid in Experimental Example 2.
[FIG. 6] is a view showing the results of all drug-organoid combinations in high-throughput screening (HTS) carried out in Experimental Example 2.
[FIG. 7] is a graph showing the distribution of Z'-factors in 237 screening plates in Experimental Example 3.
[FIG. 8] is a graph showing a root mean square error (r.m.s.e) of a sigmoid response curve fitted to a dose-response curve in Experimental Example 3.
[FIG. 9] is a graph showing the results of comparing $\log_2 (IC_{50})$ of 56 types of drugs for a normal colon organoid line (NLCRC15) acquired in triplicate in Experimental Example 3 between repeated experiments.

[FIG. 10] is a graph showing the results of calculating Spearman's correlation coefficients for all pairs of organoid lines based on values of $IC_{50}$ in Experimental Example 3.

[FIG. 11] is a volcano plot showing the results of multivariate analysis of variance (MANOVA) in Experimental Example 4.

[FIG. 12] is a graph showing the $IC_{50}$ of (+)-JQ1 for a normal colon organoid (n = 6) and a CRC organoid (n = 15), measured in Experimental Example 4.

[FIG. 13] is a set of representative micrographs showing a response of a normal colon organoid and a CRC organoid to (+)-JQ1, acquired in Experimental Example 4. The scale bar is 500 $\mu$m.

DESCRIPTION OF EMBODIMENTS

[Production method for organoid]

In one embodiment, the present invention provides a production method for an organoid, including a step of dissociating a biological tissue and a step of carrying out suspension culture of the dissociated biological tissue in a state of being dispersed in a medium to form an organoid, in which the medium is substantially free of a p38 inhibitor and contains the following components (i) to (iv).

(i) 0.1% to 10% by volume of an extracellular matrix
(ii) IGF-1
(iii) FGF-2
(iv) at least one selected from the group consisting of a Wnt agonist, a BMP inhibitor, a TGF-$\beta$ inhibitor, and an EGF

As will be described later in Examples, the production method according to the present embodiment makes it possible to carry out stable large-scale culture of a normal tissue-derived organoid and to achieve large-scale production of the normal tissue-derived organoid. The production method according to the present embodiment can also be said to be a culture method for an organoid. In the present embodiment, the large-scale culture means, for example, proliferating organoids of $10^6$ to $10^7$ cells from a tissue derived from a living body.

In the production method according to the present embodiment, the biological tissue may be a biological tissue derived from a patient, or may be an organoid which has already been established. In a case where the biological tissue is an organoid, the production method according to the present embodiment can also be said to be a subculture method for an organoid.

In the production method according to the present embodiment, the biological tissue is preferably an epithelial tissue. Examples of the epithelial tissue include an intestinal epithelial tissue. The intestinal tract is an organ in the human body that has the largest area in contact with the external environment, and has functions essential for maintaining life such as digestion and absorption. Most of the functions of the intestinal tract are carried out by the intestinal epithelium that covers an inner layer of the intestinal tract. The intestinal epithelium is composed of two compartments: villi, which consist of three lineages of differentiated cells (mucus-producing cells, absorptive epithelial cells, and endocrine cells), and crypts, which mainly consist of undifferentiated proliferative cells. The epithelial tissue is preferably a tissue containing epithelial stem cells or epithelial cells. The epithelial cells include differentiated epithelial cells and epithelial stem cells. The "epithelial stem cell" refers to a cell having a long-term self-replication function and an ability to differentiate into an epithelial differentiated cell, and means a stem cell derived from an epithelial tissue. Examples of the epithelial tissue include tissues of cornea, oral mucosa, skin, conjunctiva, bladder, renal tubule, kidney, digestive organs (esophagus, stomach, duodenum, small intestine (including jejunum and ileum), and large intestine (including colon)), liver, pancreas, mammary gland, salivary gland, tear gland, prostate, hair root, trachea, lung, and the like.

The biological tissue is preferably a tissue derived from a human or non-human mammal. The non-human mammal includes, but is not particularly limited to, a mouse, a rat, a cow, a pig, a rabbit, a monkey, and the like. In the production method according to the present embodiment, the biological tissue may be a tumor tissue or a cancer tissue, or may be a normal tissue. Here, the tumor tissue means a tissue that has become tumorous, and the cancer tissue means a tissue that has become cancerous.

Dissociating a biological tissue means fragmenting the biological tissue and dividing the fragmented biological tissues into cell clusters each consisting of one cell to several cells. Here, "several" may mean about 2 to 100 cells. The dissociation of the biological tissue may be carried out by mechanical shearing or by an enzymatic treatment with collagenase, dispase I, liberase, trypsin, TrypLE Express (available from Thermo Fisher Scientific, Inc.), or the like.

It is known that the organoid may have poor recovery after being dissociated into a single cell. On the other hand, the present inventors have found that a p38 inhibitor, which has recently been considered to be essential for culturing human intestinal organoids, inhibits the growth of organoids from single cells. The present inventors further found that replacing the p38 inhibitor with a combination of IGF-1 and FGF-2 promoted the growth of organoids from single cells.

**In** addition, general organoid culture protocols in the related art include a step of embedding the dissociated organoid in an extracellular matrix such as Matrigel (registered trademark). However, the organoid embedded in the extracellular matrix may be difficult to scale up in culture. **In** addition, the organoid embedded in the extracellular matrix may exhibit a large variation in growth.

On the other hand, the production method according to the present embodiment is carried out in such a manner that the dissociated biological tissue is subjected to suspension culture in a state of being dispersed in a medium. That is, in the production method according to the present embodiment, the dissociated biological tissue is not embedded in an extracellular matrix, but is subjected to suspension culture in a state in which cells are dispersed in a medium containing an extracellular matrix.

As will be described later in Examples, the production method according to the present embodiment makes it possible to achieve stable large-scale culture of not only a cancer organoid but also a normal tissue-derived organoid. In addition, by accurately aligning the number of cells at the time of seeding the cells, it is possible to suppress the variation in the number of cells among wells. In order to align the number of cells, for example, sorting by a cell sorter can be used.

Examples of p38 inhibitors that have been used in the culture of organoids in the related art include SB202190 (CAS number: 152121-30-7), SB203580 (CAS number: 152121-47-6), VX-702 (CAS number: 745833-23-2), VX-745 (CAS number: 209410-46-8), PD169316 28CAS number: 152121-53-4), RO4402257 (CAS number: 1449811-01-2), and BIRB796 (CAS number: 285983-48-4).

On the other hand, the production method according to the present embodiment uses a medium that is substantially free of a p38 inhibitor. In the present specification, the phrase "substantially free of a p38 inhibitor" means that no p38 inhibitor is intentionally added to a medium, and means that the unavoidable incorporation of the p38 inhibitor is permitted within a range in which the effects of the present embodiment are exhibited.

The medium used in the production method according to the present embodiment contains an extracellular matrix. The extracellular matrix is a non-cellular component and includes various polysaccharides, water, elastins, and glycoproteins. Examples of the glycoproteins include collagen, entactin (nidogen), fibronectin, and laminin.

A commercially available extracellular matrix may be used as the extracellular matrix. Examples of the commercially available extracellular matrix include an extracellular matrix protein (available from Thermo Fisher Scientific, Inc.), a basement membrane preparation derived from Engelbreth-Holm-Swarm (EHS) mouse sarcoma cells (for example, Matrigel (registered trademark), available from Corning Incorporated), and ProNectin (available from Sigma-Aldrich Co. LLC).

The medium used in the production method according to the present embodiment preferably contains 0.1% to 10% by volume, for example, 0.1% to 5% by volume, for example, 0.1% to 3% by volume, or for example, about 2% by volume, of an extracellular matrix.

The medium used in the production method according to the present embodiment contains IGF-1. IGF-1, also known as somatomedin C, is a factor secreted primarily in the liver in response to stimulation by growth hormone (GH). The concentration of IGF-1 contained in the medium may be, for example, 5 ng/mL to 1 $\mu$g/mL, for example, 10 ng/mL to 1 $\mu$g/mL, and for example, 50 to 500 ng/mL.

The medium used in the production method according to the present embodiment contains FGF-2. FGF-2 is a basic fibroblast growth factor, also called bFGF. The concentration of FGF-2 contained in the medium may be, for example, 5 ng/mL to 1 $\mu$g/mL, for example, 10 ng/mL to 1 $\mu$g/mL, and for example, 50 to 500 ng/mL.

As will be described later in Examples, the growth of organoids from single cells is promoted by replacing a p38 inhibitor, which has been considered to be essential for the culture of organoids in the related art, with a combination of IGF-1 and FGF-2. As a result, it is possible to achieve stable large-scale culture of not only a cancer organoid but also a normal tissue-derived organoid.

The medium used in the production method according to the present embodiment contains at least one selected from the group consisting of a Wnt agonist, a BMP inhibitor, a TGF-$\beta$ inhibitor, and EGF. The medium used in the production method according to the present embodiment may contain two selected from the group consisting of a Wnt agonist, a BMP inhibitor, a TGF-$\beta$ inhibitor, and EGF, may contain three selected from the group consisting of a Wnt agonist, a BMP inhibitor, a TGF-$\beta$ inhibitor, and EGF, or may contain a Wnt agonist, a BMP inhibitor, a TGF-$\beta$ inhibitor, and EGF.

The Wnt agonist refers to a substance that activates T-cell factor (hereinafter, sometimes referred to as "TCF")/lymphoid enhancer factor (hereinafter, sometimes referred to as "LEF")-mediated transcription in a cell. The Wnt agonist is not limited to a Wnt family protein, and includes an agonist that binds to and activates members of the Frizzled receptor family, an inhibitor of intracellular $\beta$-catenin degradation, and an activator of TCF/LEF.

The Wnt agonist is preferably at least one selected from the group consisting of a Wnt protein, an R-spondin, and a GSK-3$\beta$ inhibitor.

The Wnt agonist is a substance that stimulates Wnt activity in a cell by at least 10%, preferably at least 20%, more preferably at least 30%, still more preferably at least 50%, particularly preferably at least 90%, and most preferably 100% of the level of Wnt activity in the presence of the Wnt agonist, as compared to the level of Wnt activity in the absence of the Wnt agonist. The Wnt activity can be evaluated, for example, by measuring a transcriptional activity of Wnt by a TCF reporter assay.

The Wnt agonist is preferably a combination of a Wnt protein and an R-spondin. Where the Wnt protein is derived from is not particularly limited, and Wnt proteins derived from various organisms can be used. Above all, a mammal-derived Wnt protein is preferable. The mammal is the same as described hereinbefore. Examples of the mammalian Wnt protein include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16. One type of Wnt protein may be used alone, or two or more types of Wnt proteins may be used in combination.

The Wnt protein may be a Wnt protein prepared using Wnt protein-expressing cells, or may be a commercially available Wnt protein. The Wnt protein may be a fragment of the Wnt protein or may be a Wnt protein containing an amino acid sequence other than the amino acid sequence of the Wnt protein as long as it has a Wnt activity. Examples of the amino acid sequence other than the amino acid sequence of the Wnt protein include an amino acid sequence of an affinity tag. In addition, the amino acid sequence of the Wnt protein does not need to be completely identical to an amino acid sequence that is available from a known database such as GenBank, and may be an amino acid sequence in which one to several amino acids have been deleted, substituted, or added in the amino acid sequence that is available from a known database, as long as it has a Wnt activity. Here, the number of from one to several is preferably 10 or less, more preferably 7 or less, and still more preferably 6 or less.

The concentration of the Wnt protein contained in the medium may be, for example, 50 ng/mL to 10 $\mu$g/mL, for example, 100 ng/mL to 10 $\mu$g/mL, for example, 200 ng/mL to 1 $\mu$g/mL, and for example, 300 ng/mL to 1 $\mu$g/mL.

It is preferable that the Wnt protein forms a complex with afamin. The afamin is a glycoprotein that belongs to an albumin family and is known to be present in body fluids such as blood. Serum added to a cell culture medium contains afamin derived from an animal from which the serum was collected. The serum contains impurities or the like other than afamin, so it is preferable to use afamin alone without using the serum.

Where the afamin is derived from is not particularly limited, and afamin derived from various organisms can be used. Above all, mammal-derived afamin is preferable. The mammal is the same as described hereinbefore.

The afamin may be natural afamin contained in serum or the like, which is purified by a known method, or may be recombinant afamin. The recombinant afamin can be produced by appropriately using a known genetic recombination technology. The recombinant afamin can be produced, for example, by inserting DNA encoding afamin into a known expression vector, introducing the resulting expression vector into an appropriate host cell to express recombinant afamin, and purifying the recombinant afamin using a known purification method.

The recombinant afamin may be afamin with an affinity tag added. The affinity tag to be added is not particularly limited and can be appropriately selected from known affinity tags and then used. The affinity tag is preferably an affinity tag which is recognized by a specific antibody, and examples thereof include a FLAG tab, an MYC tag, an HA tag, and a V5 tag.

The above-mentioned Wnt protein has strong hydrophobicity because a specific serine residue is modified with a fatty acid (palmitoleic acid). Therefore, it is widely known that the Wnt protein is prone to aggregation or denaturation in an aqueous solution, making purification and storage of the Wnt protein extremely difficult. Meanwhile, it has been reported that the modification of this specific serine residue with a fatty acid is essential for the physiological activity of the Wnt protein and is involved in binding to members of the Frizzled receptor family.

The Wnt protein binds to afamin on a one-to-one basis to form a complex in an aqueous solution and can therefore be solubilized while maintaining a high physiological activity. Therefore, a Wnt protein-afamin complex may be produced by culture of cells expressing both a Wnt protein and afamin, or a Wnt protein-afamin complex may be produced by co-culture of Wnt protein-expressing cells and afamin-expressing cells. The Wnt protein-afamin complex may be added to the medium in the form of a conditioned medium.

The concentration of afamin contained in the medium may be, for example, 50 ng/mL to 10 $\mu$g/mL, for example, 100 ng/mL to 10 $\mu$g/mL, for example, 200 ng/mL to 1 $\mu$g/mL, and for example, 300 ng/mL to 1 $\mu$g/mL.

Examples of the R-spondin include the R-spondin family consisting of R-spondin 1, R-spondin 2, R-spondin 3, and R-spondin 4. The R-spondin family is a secretory protein known to be involved in the activation and regulation of the Wnt signaling pathway. The R-spondin to be used may be a fragment of the R-spondin or an R-spondin containing an amino acid sequence other than the amino acid sequence of the R-spondin, such as an affinity tag, as long as it has an R-spondin activity. One type of R-spondin may be used alone, or two or more types of R-spondins may be used in combination.

The concentration of R-spondin contained in the medium may be, for example, 50 ng/mL to 10 $\mu$g/mL, for example, 100 ng/mL to 10 $\mu$g/mL, for example, 200 ng/mL to 1 $\mu$g/mL, and for example, 300 ng/mL to 1 $\mu$g/mL. The R-spondin may be added to the medium in the form of a conditioned medium.

Examples of the GSK-3$\beta$ inhibitor include CHIR99021 (CAS number: 252917-06-9), kenpaullone (CAS number: 142273-20-9), and 6-bromoindirubin-3'-oxime (BIO, CAS number: 667463-62-9).

Examples of the BMP inhibitor include substances having an inhibitory activity of preferably 50% or more, more preferably 70% or more, still more preferably 80% or more, and particularly preferably 90% or more, as compared with the level of BMP activity in the absence of the inhibitor. The BMP inhibitory activity can be evaluated by measuring the transcriptional activity of BMP.

The BMP inhibitor is preferably a naturally occurring BMP-binding protein, examples of which include a chordin-like protein such as noggin, gremlin, chordin, or a chordin domain; a follistatin-related protein such as follistatin or a follistatin domain; a DAN-like protein such as a differential screening-selected gene aberrative in neuroblastoma (DAN) or a DAN cysteine-knot domain; sclerostin (SOST); decorin; and α-2-macroglobulin.

The concentration of the BMP inhibitor contained in the medium may be, for example, 10 ng/mL to 1 μg/mL, for example, 20 ng/mL to 1 μg/mL, for example, 50 ng/mL to 1 μg/mL, and for example, about 100 ng/mL.

TGF-β is a type of growth factor and is produced in almost all cells of the kidney, bone marrow, platelets, and the like. There are five subtypes (β1 to β5) in TGF-β. In addition, it is known that TGF-β promotes the proliferation of osteoblasts and the synthesis and proliferation of connective tissues such as collagen and exhibits an inhibitory effect against epithelial cell proliferation and osteoclasts. In general, the TGF-β inhibitor is, for example, a drug that blocks or inhibits the binding of TGF-β to a TGF-β receptor and binds to TGF-β to form a complex which neutralizes the TGF-β activity. In addition, the TGF-β inhibitor is, for example, a drug that binds to a TGF-β receptor to block or inhibit the binding of TGF-β to the TGF-β receptor and acts as an antagonist or inverse agonist.

Examples of the TGF-β inhibitor include substances having an inhibitory activity of preferably 50% or more, more preferably 70% or more, still more preferably 80% or more, and particularly preferably 90% or more, as compared with the level of TGF-β activity in the absence of the inhibitor.

Examples of the TGF-β inhibitor include A83-01 (CAS number: 909910-43-6), SB-431542 (CAS number: 301836-41-9), SB-505124 (CAS number: 694433-59-5), SB-525334 (CAS number: 356559-20-1), LY364947 (CAS number: 396129-53-6), SD-208 (CAS number: 627536-09-8), and SJN2511 (CAS number: 446859-33-2). Among these, A83-01 is preferable.

The concentration of the TGF-β inhibitor contained in the medium may be, for example, 100 nM to 1 μM, for example, 200 nM to 1 μM, for example, 300 nM to 1 μM, or for example, about 500 nM.

In the production method according to the present embodiment, the medium may contain a mitogenic growth factor in addition to the above-mentioned components. Examples of the mitogenic growth factor include an epidermal growth factor (EGF), a transforming growth factor-α (TGF-α), and a keratinocyte growth factor (KGF). These mitogenic growth factors may be used alone or in combination of two or more thereof.

EGF is a potent mitogen for a variety of cultured ectodermal cells and mesodermal cells and has a significant effect on the differentiation of some fibroblasts and specific cells. The EGF precursor exists as a membrane-bound molecule that is proteolytically cleaved to generate a 53-amino acid peptide hormone that stimulates cells.

Above all, EGF is preferable as the mitogenic growth factor. The concentration of EGF contained in the medium may be, for example, 5 to 500 ng/mL, for example, 10 to 400 ng/mL, and for example, about 50 ng/mL.

In the production method according to the present embodiment, it is preferable that the suspension culture of the dissociated biological tissue is carried out while stirring the medium. In addition, as will be described later in Examples, in a case of carrying out the suspension culture with stirring, the organoids can be proliferated more rapidly than in a case of the suspension culture.

[Screening method for anticancer drug]

In one embodiment, the present invention provides a screening method for an anticancer drug, including a step of carrying out suspension culture of a normal tissue-derived organoid and a tumor tissue-derived organoid in a state of being dispersed in a medium in the presence of a test substance; and a step of evaluating an effect of the test substance on the normal tissue-derived organoid and the tumor tissue-derived organoid, in which the test substance that has few side effects on the normal tissue-derived organoid and is effective on the tumor tissue-derived organoid is shown to be an anticancer drug that is effective against the tumor tissue, and the medium is substantially free of a p38 inhibitor and contains the following components (i) to (iv).

    (i) 0.1% to 10% by volume of an extracellular matrix
    (ii) IGF-1
    (iii) FGF-2
    (iv) at least one selected from the group consisting of a Wnt agonist, a BMP inhibitor, a TGF-β inhibitor, and an EGF

In the related art, it has been difficult to achieve stable large-scale culture of a normal tissue-derived organoid, and thus it has not been possible to incorporate the normal tissue-derived organoid into drug screening. For this reason, it has been difficult to evaluate side effects and safety of a drug on normal tissues.

On the other hand, as will be described later in Examples, the screening method according to the present embodiment makes it possible to apply normal tissue-derived organoids, along with tumor tissue-derived organoids, to drug screening. As a result, it is possible to evaluate side effects and safety of a drug on normal tissues. In addition, as will be described later in Examples, it is possible to screen for a drug that specifically suppresses the growth of tumor tissue-derived organoids without affecting the proliferation of normal tissue-derived organoids.

As will be described later in Examples, by accurately aligning the number of cells at the time of seeding organoids, it is possible to suppress the variation in the number of cells among wells. In order to align the number of cells, for example, sorting by a cell sorter can be used.

In the screening method according to the present embodiment, the test substance is not particularly limited, and for example, a natural compound library, a synthetic compound library, or an existing drug library can be used. The screening method according to the present embodiment makes it possible to carry out high-throughput screening (HTS) having high accuracy, robustness, and reproducibility.

In the screening method according to the present embodiment, the normal tissue-derived organoid is preferably an organoid produced by the above-mentioned production method. In the screening method according to the present embodiment, the extracellular matrix, IGF-1, FGF-2, Wnt agonist, BMP inhibitor, TGF-β inhibitor, EGF, organoid, and the like are the same as those described hereinbefore.

Examples

Hereinafter, the present invention will be described in more detail by way of Examples; however, the present invention is not intended to be limited to the following Examples.

[Material and method]

(Establishment of patient-derived organoid)

Seven newly established organoid lines and patient-derived organoid lines established in a previous study were used. All samples used for organoid establishment and biological analysis were obtained from Keio University Hospital or the University of Tokyo Hospital with written informed consent. The present study was approved by the ethics committees of both research institutes.

Normal colon tissues and neoplastic colon tissues were obtained from any of endoscopic biopsies or surgically resected specimens. Samples were stored in ice-cold phosphate buffered saline (PBS) until the treatment. Fresh tissue samples were cut into small pieces, washed at least 10 times with ice-cold PBS, and then digested with liberase (TH grade, available from F. Hoffmann-La Roche Ltd.) at 37°C for 60 min while being vigorously pipetted every 15 min. The remaining fragments were further treated with TrypLE Express (available from Thermo Fisher Scientific, Inc.) at 37°C for 20 min. Subsequently, the supernatant was collected and centrifuged at $200 \times$ g for 3 min at 4°C. Subsequently, the cell pellet was suspended in Matrigel (registered trademark) (growth factor reduced, available from BD Biosciences) and dispensed into a 48-well culture plate (25 μL of Matrigel (registered trademark) per well). The names of the organoid lines used and the properties of the tissues from which the organoids were derived are shown in Table 1 below. In Table 1, in the column of "Histology", "NL" indicates a normal tissue, "TA" indicates a tubular adenoma, "SSL" indicates a serrated lesion, "well" indicates a well-differentiated adenocarcinoma, "mod" indicates a moderately differentiated adenocarcinoma, "por" indicates a poorly differentiated adenocarcinoma, and "muc" indicates a mucinous adenocarcinoma. In addition, in the column of "Position", "A" indicates the ascending colon, "T" indicates the transverse colon, "D" indicates the descending colon, "S" indicates the sigmoid colon, "R" indicates the rectum, "LM" indicates liver metastasis, and "C" indicates the cecum. In addition, in the column of "Gender", "F" indicates a female and "M" indicates a male. In addition, in the column of "Culture conditions", "W" indicates Wnt, "E" indicates EGF, "N" indicates noggin, "R" indicates R-Spondin1, "A" indicates A83-01, "S" indicates SB202190, and "-" indicates that no growth factor is required.

[Table 1]

| Organoid name | Histology | Position | Gender | Age | Stage | Culture conditions |
|---|---|---|---|---|---|---|
| NLSL1 | NL | A | F | 68 | - | WENARFI |
| NLCRC15 | NL | T? | F | 84 | - | WENARFI |
| NLCRC25 | NL | T? | M | 50 | - | WENARFI |
| NLCRC26 | NL | ? | F | 66 | - | WENARFI |
| NLCRC32 | NL | D | M | 68 | - | WENARFI |
| NLCRC33 | NL | T | F | 74 | - | WENARFI |
| NLCRC35 | NL | R | M | 50 | - | WENARFI |

(continued)

| Organoid name | Histology | Position | Gender | Age | Stage | Culture conditions |
|---|---|---|---|---|---|---|
| NLSL3 | NL | A | M | 69 | - | WENARFI |
| Ad2 | TA | A | A | 40 | - | ENAFI |
| SL3 | SSL | A | M | 69 | - | WENRA |
| CRC5 | well | D | M | 58 | I | ENAFI |
| CRC7 | mod | S | M | 85 | II | ENA |
| CRC11 | mod | C | M | 75 | II | - |
| CRC12 | mod | A | M | 45 | II | ENA |
| CRC13 | muc | T | F | 70 | II | WENAR |
| CRC15 | por | T | F | 84 | II | ENA |
| CRC17a | por, mod | A | M | 82 | III | ENA |
| CRC17b | por, mod | A | M | 82 | III | - |
| CRC18a | muc | C | F | 70 | III | WENARAFI |
| CRC18b | muc | C | F | 70 | III | WENARAFI |
| CRC20 | mod | R | F | 67 | IV | E |
| CRC23b | mod | R | M | 72 | IV | EFI |
| CRC24b | mod | R | F | 48 | IV | ENAFI |
| CRC25a | mod | T | M | 50 | IV | ENA |
| CRC25c | mod | LM | M | 50 | IV | ENA |
| CRC28 | mod | LM | M | 63 | IV | EA |
| CRC31 | por, mod | R | M | 45 | II | ENFI |
| CRC32 | por, mod | D | M | 68 | IV | - |
| CRC33 | mod | T | F | 74 | II | WNRA |
| CRC34 | mod | R | F | 40 | IV | ENAFI |

(Culture of organoid)

Human colorectal cancer (CRC) organoids were cultured in a medium containing 2% by volume of Matrigel (registered trademark) (available from Corning Incorporated). The medium used was a medium in which a basal medium was supplemented with various combinations of growth factors depending on whether the organoid is derived from a normal tissue or a tumor tissue, and depending on the origin of the tumor in a case where the organoid is derived from a tumor tissue. In a case of stirring the medium, Orbital Shaker MaxQ 2000 CO2 Plus, CO$_2$ Resistant (available from Thermo Fisher Scientific, Inc.) was used.

The basal medium used was a medium in which an advanced DMEM/F12 was supplemented with penicillin/-streptomycin, 10 mM HEPES, 2 mM GlutaMAX, 1 × B27 (available from Thermo Fisher Scientific, Inc.), 10 nM gastrin I (available from Sigma-Aldrich Co. LLC), and 1 mM N-acetylcysteine (available from FUJIFILM Wako Pure Chemical Corporation).

The normal tissue-derived organoids were maintained in an improved medium or a conventional medium. The improved medium was a medium in which a basal medium was supplemented with the following seven types of growth factors.

20% afamin-Wnt3A serum-free conditioned medium
10% R-spondin-1 conditioned medium
50 ng/mL mouse recombinant EGF (available from Thermo Fisher Scientific, Inc.)
500 nM A83-01 (available from Tocris Bioscience)
100 ng/mL mouse recombinant noggin (available from PeproTech Inc.)
100 ng/mL recombinant human IGF-1 (available from BioLegend, Inc.)

50 ng/mL recombinant human FGF-basic (FGF-2) (available from PeproTech Inc.)

The conventional medium was the same as the improved medium, except that 10 $\mu$M SB202190 (available from Sigma-Aldrich Co. LLC) was added instead of IGF-1 and FGF-2. In the tumor organoids, some growth factors were not required due to genetic mutations.

The subculture was carried out by collecting and washing the organoids and fragmenting the organoids by mechanical shearing or digestion with TrypLE Express (available from Thermo Fisher Scientific, Inc.). For 2 days after the subculture, the fragments of the organoids were cultured in a fresh medium containing 10 $\mu$M Y-27632 (available from FUJIFILM Wako Pure Chemical Corporation) to suppress cell death.

(Drug screening assay)

The standard inhibitor kit (SCADS Inhibitor Kit) drug library was provided by the Screening Committee of Anticancer Drugs on New Academic Area Research, Cancer Research Support Project, Grant-in-Aid for Scientific Research from The Ministry of Education, Culture, Sports, Science and Technology, Japan.

In the present study, a total of 23 organoid lineages were subjected to screening. Prior to screening, the organoids were labeled with a green fluorescent protein (GFP) using PB-CMV-MCS-EF1$\alpha$-GFP-Puro PiggyBac (PB513B-1, available from System Biosciences, LLC.) and Super PiggyBac transposase (PB210-20, available from System Biosciences, LLC.) vectors.

The organoids were dissociated with TrypLE Express (available from Thermo Fisher Scientific, Inc.) and filtered using a cell strainer (pore size: 20 $\mu$m, available from Sysmex Corporation). Using a cell sorter (SH800, available from Sony Corporation), 800 to 1,500 cells were sorted into each well of a 384-well plate.

The organoids were treated with a test compound 24 hours or 96 hours after seeding. The organoids were grown for 6 to 8 days, and the colony area was measured based on the fluorescence of GFP using an IN Cell Analyzer 6000 confocal imaging system (available from GE Healthcare Life Sciences). In addition, bright field imaging and luminescence-based ATP measurement were carried out using Cell3iMager duos (available from SCREEN Holdings Co., Ltd.) and CellTiter-Glo cell viability assay (available from Promega Corporation).

The bright field image was analyzed using software integrated into the Cell3iMager. Default parameters were used for the pretreatment and the object detection, and supervised viability determination was applied to the machine learning. In the evaluation assay of (+)-JQ1, the cell viability was determined using a CellTiter-Glo cell viability assay (available from Promega Corporation).

(Dose-response curve fitting)

The dose-response curve was fitted to the drug response data using a Bayesian sigmoid model. The drug response data was obtained in triplicate as drug responses at 7 points at a 2-fold concentration, including 6 or more drug-free controls. In all the screening plates, the proteasome inhibitor MG-132 was tested at a maximum concentration of 1 $\mu$M, giving 100% cell death and an accurate estimation of the background signal.

The sigmoid response curve of 4 parameters was fitted as follows. First, the viability of the organoid was normalized by dividing by the average of the drug-free controls. The viability $X_{lc}$ of the organoid was estimated to be an average value of Expression (1).

$$X_{lc} = I_{min} + (I_{max} - I_{min})/(1 + e^{(lc - \beta pos)/\beta shape}) \ldots (1)$$

In Expression (1), $I_{max}$ represents a viability of a drug-free control, $I_{min}$ represents an average of background signals, $\beta pos$ represents a position of a sigmoid curve, and $\beta shape$ represents steepness. 1c represents the log concentration.

It was assumed that the viability $X_{lc}$ of the organoid has a gamma distribution with variance $Var(X_{lc}) = BE(X_{lc})$. Here, B represents a noise parameter. It was also assumed that the viability $X_{max}$ of the drug-free control has a gamma distribution with variance $Var(X_{max}) = BI_{max}$.

The Markov chain Monte Carlo simulation was carried out using rstan (v.2.18.0) to estimate the parameters. The initial values were randomly generated from the prior distribution. Following 1,000 burn-in iterations, 1,000 iterations were sampled from four independent chains to obtain a posterior distribution. The median of the posterior distribution was used as the parameter estimate value. $\beta pos$ had a normal prior distribution with a 99% probability mass covering a range from 1/100 of the tested minimum concentration to 100 times the tested maximum concentration. $I_{max}$ had a uniform prior distribution between 0 and 5. $I_{min}$ was reparameterized as $I_{min} = \alpha I_{max}$ where $\alpha$ has a uniform prior distribution between 0 and 1. B and $\beta shape$ had low-informative prior probabilities (half-Cauchy distribution with location = 0, scale = 10). In a case where the R convergence metric was > 1.1 or in a case where the average absolute error was > 0.3, the curve fitting was deemed insufficient and discarded without further downstream analysis.

AUC values were estimated using trapezoidal approximation. The AUC values were determined as a part of a rectangle that covers the tested concentration range and a region surrounded by $I_{max}$ and $I_{min}$ in a vertical direction.

[Experimental Example 1]

(Suspension culture of organoid)

Human colorectal cancer (CRC) organoids were subjected to suspension culture in a medium supplemented with 2% by volume of Matrigel (registered trademark), and the proliferation thereof was examined. For the purpose of comparison, the culture was also carried out by embedding the organoids in droplets of Matrigel (registered trademark), which is a culture method in the related art. In addition, suspension culture with stirring, which involves suspension culture of the organoids and stirring of the medium, was also carried out.

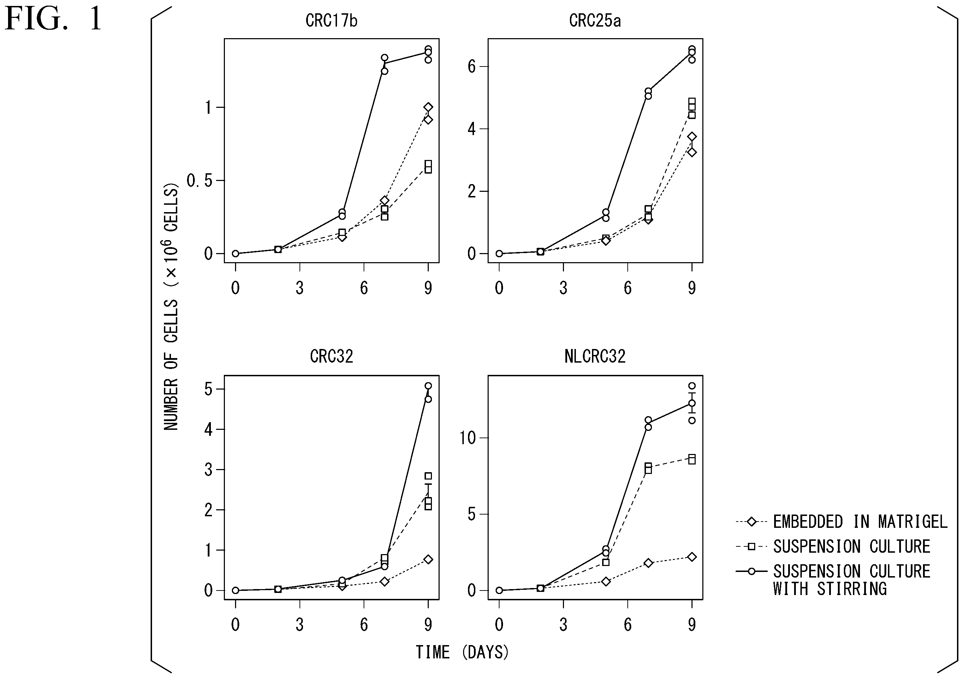

FIG. 1 is a graph showing the results of measuring the proliferation of organoids under each culture condition (n = 3). In FIG. 1, "CRC17b", "CRC25a", "CRC32", and "NLCRC32" are the names of organoid lines. The properties of each organoid line are shown in Table 1 above. CRC17b, CRC25a, and CRC32 are CRC organoids, and NLCRC32 is a normal tissue-derived organoid. As a result, it was revealed that in any of the organoids, the organoids more rapidly proliferated to a scale of $10^6$ to $10^7$ cells in a case of carrying out the suspension culture than in a case of carrying out the culture embedded in droplets of Matrigel (registered trademark). In addition, in a case of carrying out the suspension culture with stirring, the organoids proliferated even more rapidly.

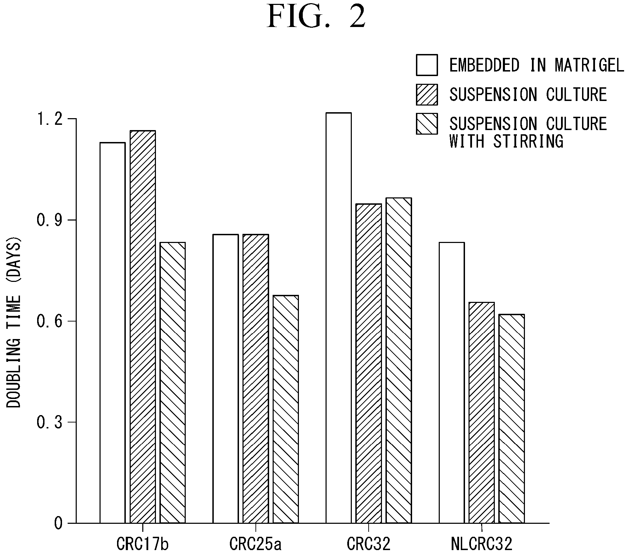

FIG. 2 is a graph showing the results of calculating a doubling time of the organoids under each culture condition. As a result, it was found that, compared to the culture embedded in droplets of Matrigel (registered trademark), the suspension culture tends to have a shorter doubling time, and the suspension culture with stirring tends to have a further shorter doubling time.

[Experimental Example 2]

(Robust high-throughput screening (HTS) system for patient-derived colorectal organoids)

In a case where the organoids are unevenly dissociated, particularly, in a case where the organoids are dissociated by mechanical shearing, selective proliferation of organoids occurs from a large fragment of the organoid, leading to variation in the proliferation of the organoids among wells.

Therefore, in order to accurately distribute the same number of living single cells per one culture well, the organoids were labeled with GFP and sorted into individual wells of a 384-well plate by flow cytometric cell sorting. Fluorescent labeling of the organoids also has made it become possible to carry out automated imaging of the organoids and measurement of the proliferation of the organoids using a high-content cell image analyzer.

Using a drug library containing 56 types of drugs, the drug sensitivity of CRC organoids and normal colon organoids to each drug was analyzed. The drug library included 9 types of anticancer drugs and 47 types of low-molecular-weight inhibitors targeting cancer-related signaling pathways, which are used in clinical practice. Among the organoid lines shown in Table 1 above, 6 normal colon organoid lines and 20 CRC organoid lines were subjected to HTS. HTS was carried out with a total of 1,438 types of drug-organoid combinations using 237 screening plates.

Drugs from the drug library were added 4 days after seeding the cells, and the proliferation of the organoids was calculated based on the area of the fluorescent dye. In order to confirm that the results obtained by this method are comparable to the results obtained by other methods, the viability of organoids was also analyzed by an ATP-based luminescence assay and bright field imaging analysis.

Fluorescence measurement and ATP-based luminescence assay showed similar results for many drugs. In addition, the bright field imaging analysis also gave results similar to those of the ATP-based luminescence assay. However, morphology varied among the CRC organoids, so the determination of whether the cells are alive or dead by the bright field imaging analysis required post hoc parameter calibration for each organoid line. For this reason, the determination of whether the cells are alive or dead by the bright field imaging analysis tends to impair the ease of screening and reduce the throughput.

HTS using human normal colon organoids has been difficult to carry out in the related art due to poor recovery of the human normal colon organoids after dissociation thereof into single cells. On the other hand, the present inventors have found that a p38 inhibitor, which has recently been considered to be essential for culturing human intestinal organoids, inhibits the growth of organoids from single cells. The present inventors also found that replacing the p38 inhibitor with a combination of IGF-1 and FGF-2 promoted the growth of organoids from single cells.

Organoids were cultured in a conventional medium containing a p38 inhibitor (SB202190) and in an improved medium containing IGF-1 and FGF-2 instead of the p38 inhibitor, and the proliferation of the organoids was measured based on the area of the fluorescent dye. 800 cells of each type were sorted and cultured in a 384-well plate.

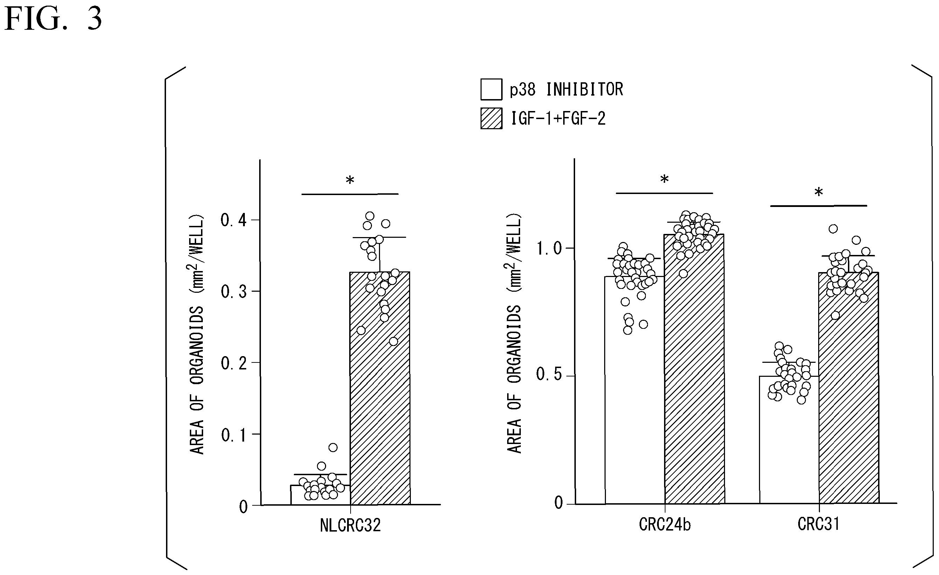

FIG. 3 is a graph showing the results of measuring the proliferation of an organoid. The left side of FIG. 3 shows the results for the normal colon organoid, and the right side of FIG. 3 shows the results for the CRC organoid. In FIG. 3, each dot indicates the measurement result for one well. The bars in the graph indicate the average value $\pm$ standard deviation. In FIG. 3, "*" indicates that there is a significant difference at $P = 9.51 \times 10^{-13}$ (NLCRC32 line, n = 22 wells), $P = 1.12 \times 10^{-21}$ (CRC24b line, n = 44 wells), and $P = 4.52 \times 10^{-21}$ (CRC31 line, n = 36 wells).

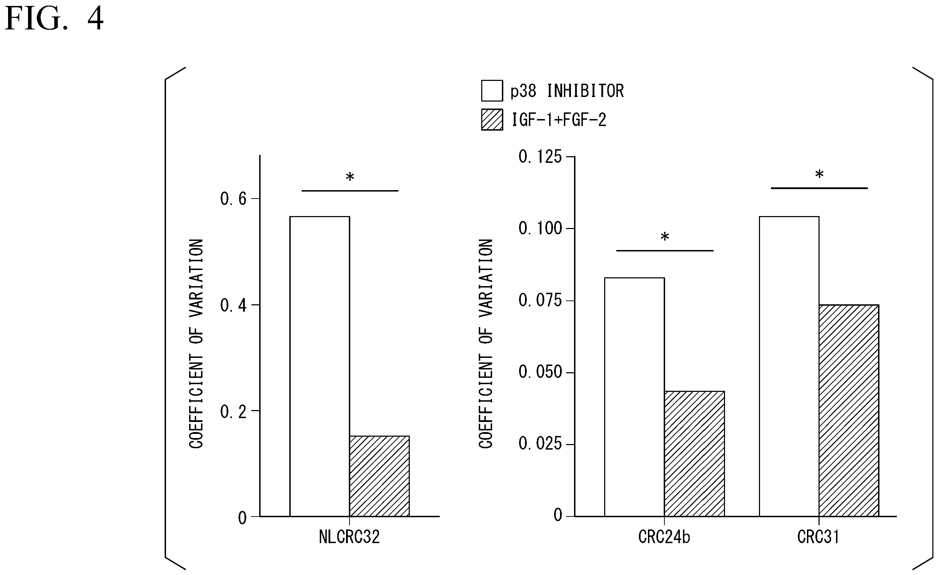

FIG. 4 is a graph showing the results of calculating a coefficient of variation of an area of a fluorescent dye. The left side of FIG. 4 shows the results for the normal colon organoid, and the right side of FIG. 4 shows the results for the CRC organoid. In FIG. 4, "*" indicates that there is a significant difference at $P = 7.28 \times 10^{-8}$ (NLCRC32 line, n = 22 wells), $P = 5.69 \times 10^{-5}$ (CRC24b line, n = 44 wells), and $P = 3.95 \times 10^{-2}$ (CRC31 line, n = 36 wells).

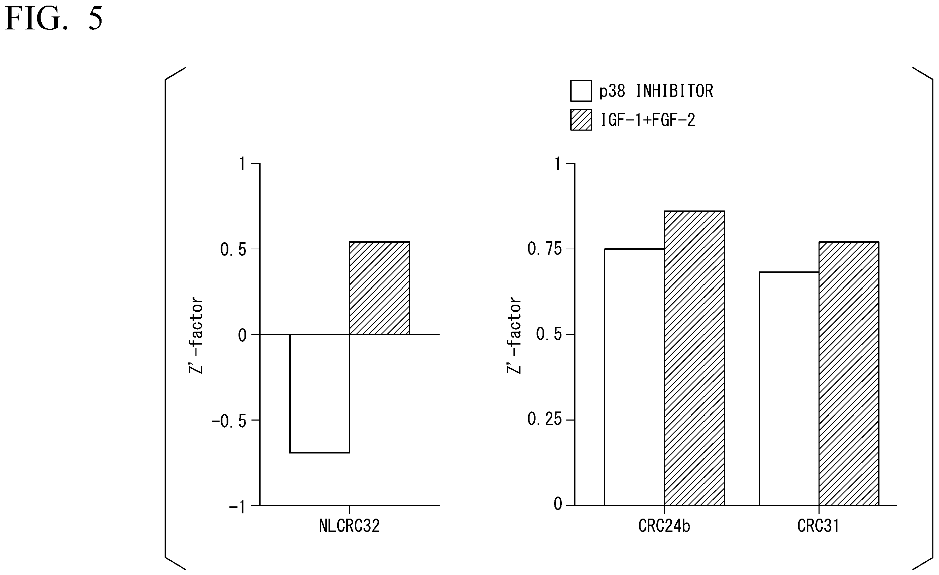

FIG. 5 is a graph showing the results of calculating a Z'-factor for each organoid. As a result, it was revealed that application of the improved medium makes it possible to achieve not only improved culture efficiency of the organoids, but also uniform culture of even normal colon organoids that had been sorted into single cells. In addition, since the Z'-factor was higher than 0.5, it was demonstrated that the present screening system was a high-quality HTS system.

The above results demonstrate that the drug screening of various organoids including a normal tissue-derived organoid can be carried out by the HTS system of the present experimental example.

[Experimental Example 3]

(Overview of drug response in colorectal organoid)

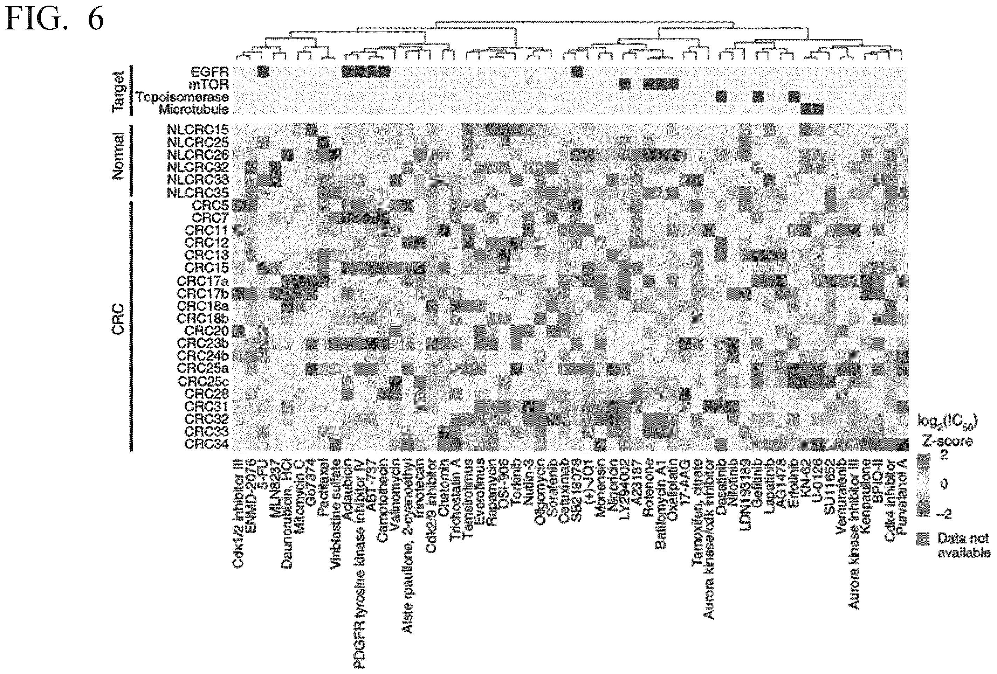

FIG. 6 is a view showing the results of all drug-organoid combinations in HTS carried out in Experimental Example 2. In FIG. 6, drug responses are shown as Z-scores of $\log_2 (IC_{50})$. Molecular targets are shown for some drugs. In HTS, 82% of the screening plates exhibited a Z'-factor greater than 0.5.

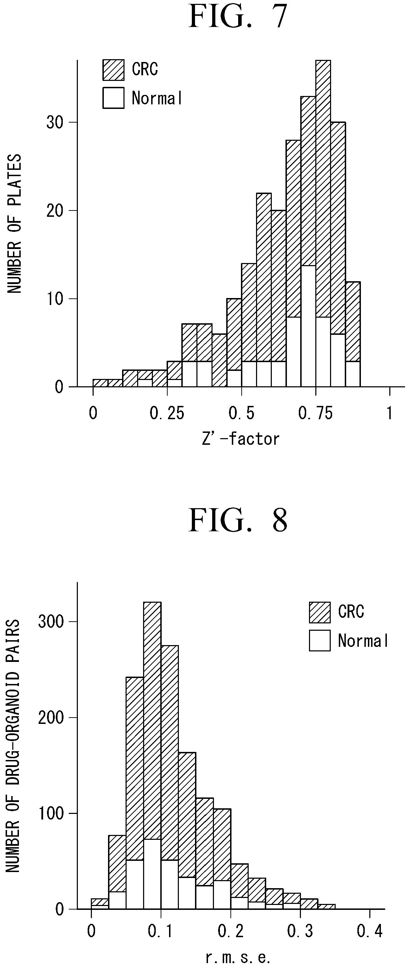

FIG. 7 is a graph showing the distribution of Z'-factors in 237 screening plates. 58 screening plates were seeded with normal colon organoids and 139 screening plates were seeded with CRC organoids. As shown in FIG. 7, no significant difference was observed in the Z'-factor between normal colon organoids and CRC organoids.

Each drug response was summarized as the 50% inhibitory concentration ($IC_{50}$), the steepness of the dose-response curve, and the area under the dose-response curve. FIG. 8 is a graph showing a root mean square error (r.m.s.e) of a sigmoid response curve fitted to a dose-response curve. The average value of the root mean square error was 0.119, indicating the robustness of the curve fitting.

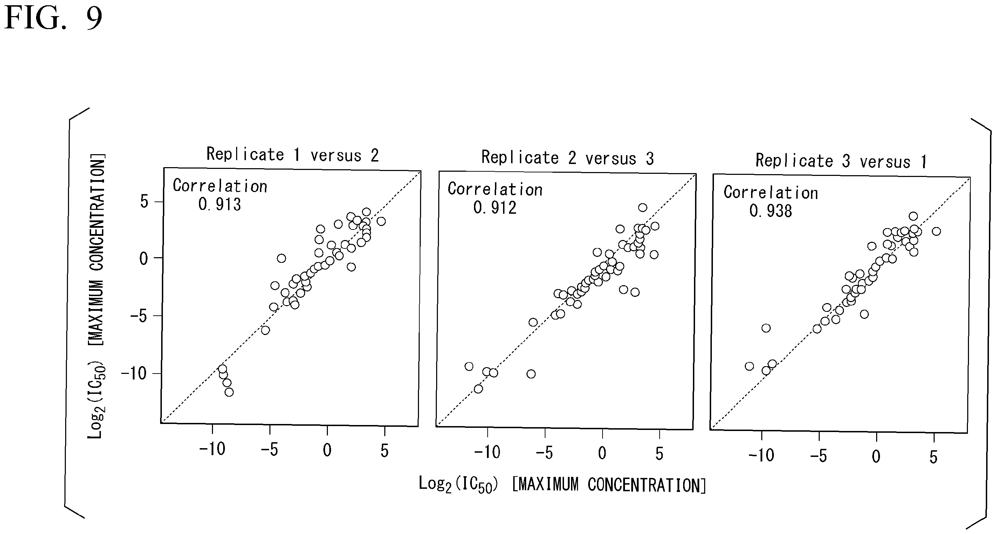

FIG. 9 is a graph showing the results of comparing $\log_2 (IC_{50})$ of 56 types of drugs for a normal colon organoid line (NLCRC15) acquired in triplicate between repeated experiments. As a result, it was confirmed that the results of three independent assays showed a high correlation.

The above results demonstrate that the HTS system of Experimental Example 2 has high accuracy, robustness, and reproducibility.

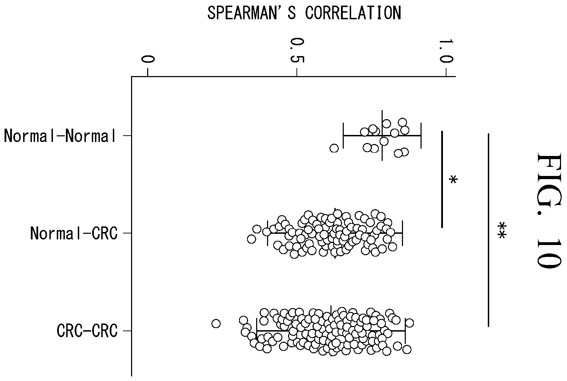

FIG. 10 is a graph showing the results of calculating Spearman's correlation coefficients for all pairs of organoid lines based on values of $IC_{50}$. In FIG. 10, each dot indicates one organoid line pair. The bars in the graph indicate the average value $\pm$ standard deviation. In addition, "*" indicates that there is a significant difference at $P = 1.34 \times 10^{-6}$, and "**" indicates that there is a significant difference at $P = 3.61 \times 10^{-7}$. In addition, "Normal-Normal" indicates the results of a pair of normal colon organoid lines (n = 15), "Normal-CRC" indicates the results of a pair of a normal colon organoid line and a CRC organoid line (n = 120), and "CRC-CRC" indicates the results of a pair of CRC organoid lines (n = 190).

As a result, the six types of normal colon organoid lines showed comparable drug responses. On the other hand, the CRC organoid lines showed significant differences in drug responses. The drug responses were significantly different between the CRC organoid lines, but as shown in FIG. 6, the CRC organoids showed similar responses to drugs that share the same targets, such as an EGFR inhibitor, an mTOR inhibitor, and a topoisomerase inhibitor.

From these results, it was considered that the heterogeneity in drug response of CRC organoids is not due to technical issues but is due to genetic or epigenetic diversity of CRC organoids.

[Experimental Example 4]

(Bromodomain and extra-terminal motif (BET) bromodomain protein inhibitor suppressed growth of CRC)

Based on the results of HTS carried out in Experimental Example 2, drug responses to normal colon organoids and CRC organoids were compared using multivariate analysis of variance (MANOVA).

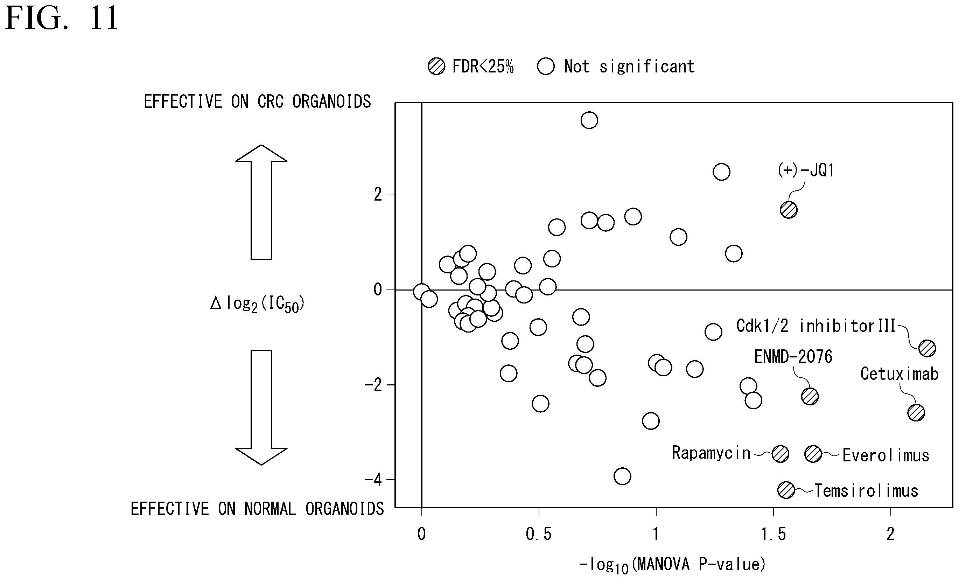

FIG. 11 is a volcano plot showing the results of MANOVA. In FIG. 11, each dot indicates one type of drug. Dots with diagonal lines indicate drugs for which a statistically significant difference was observed. More than 60% of the drugs (36/56, 64.3%) showed a higher proliferation inhibitory activity in normal colon organoids than in CRC organoids. This was thought to be due to the rapid proliferation of normal colon organoids. Despite this bias, it was shown that the BET bromodomain protein inhibitor (+)-JQ1 (CAS number: 1268524-70-4) suppressed the growth of organoids in a cancer-specific manner.

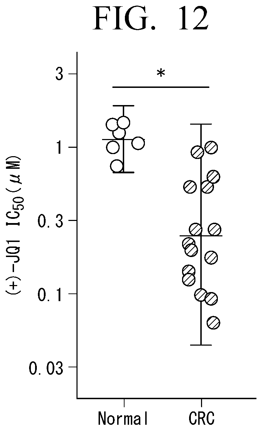

FIG. 12 is a graph showing the $IC_{50}$ of (+)-JQ1 for a normal colon organoid (n = 6) and a CRC organoid (n = 15). In FIG. 12, each dot indicates one type of organoid line. The bars in the graph indicate the average value $\pm$ standard deviation. "*" indicates that there is a significant difference at P = $1.38 \times 10^{-3}$.

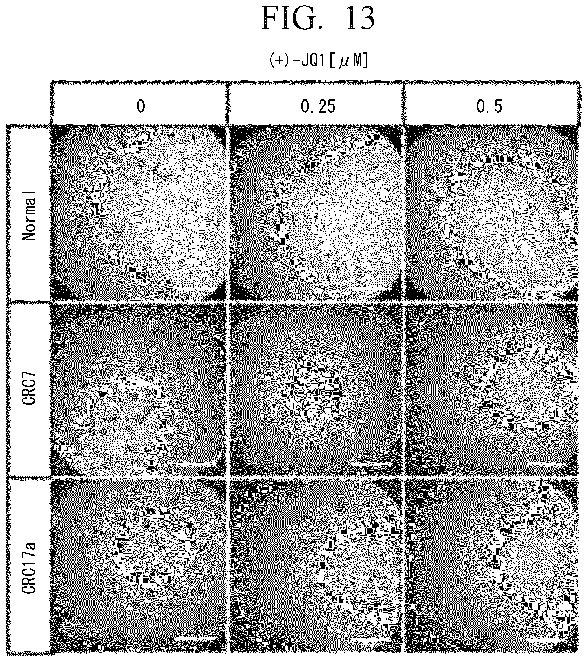

FIG. 13 is a set of representative micrographs showing a response of a normal colon organoid and a CRC organoid to (+)-JQ1. The scale bar is 500 $\mu$m. In FIG. 13, "Normal" indicates the results for normal colon organoids, and "CRC7" and "CRC17a" indicate the names of CRC organoid lines.

The above results demonstrate that it is useful to use a normal organoid together with a cancer organoid for the discovery of a potential therapeutic method.

[Example 5]

All animal experiments were carried out with the approval of the Laboratory Animal Center, Keio University School of Medicine. The verification was carried out by xenografting CRC organoids into nude mice to determine whether the results of HTS using in vitro cultured organoids were the same as those in vivo.

The CRC organoids were cultured in the same manner as in Example 1. The CRC organoids obtained by suspension culture were centrifuged, and the pellet was suspended in cold Matrigel. 0.2 mL of the CRC organoid suspension ($1 \times 10^6$ cells/injection) was transplanted with subcutaneous injection into four sites on the left and right sides of nude mice.

Tumor volumes were measured over time with an electronic caliber. After the tumor volume reached 100 to 150 $mm^3$, cetuximab (40 mg/kg, available from Merck & Co., Inc.) was intraperitoneally injected every 3 days for 7 doses. In addition, nab-paclitaxel (20 mg/kg, available from Taiho Pharmaceutical Co., Ltd.) was intravenously injected twice a week for six doses. As a result of continuing the measurement of tumor volume, it was confirmed that the results of the drug effect in vivo were in good agreement with the results of HTS obtained in vitro.

INDUSTRIAL APPLICABILITY

According to the present invention, it is possible to provide a technology that efficiently enables large-scale culture of an organoid, regardless of whether the organoid is derived from a normal tissue or derived from a tumor tissue. According to the present invention, it is possible to carry out HTS screening of drugs that have few side effects on normal tissues and are specifically effective against tumor tissues.

**Claims**

1. A production method for an organoid, comprising:

   a step of dissociating a biological tissue; and
   a step of carrying out suspension culture of the dissociated biological tissue in a state of being dispersed in a medium to form an organoid,
   wherein the medium is substantially free of a p38 inhibitor and contains the following components (i) to (iv),

   (i) 0.1% to 10% by volume of an extracellular matrix,
   (ii) insulin-like growth factor 1 (IGF-1),
   (iii) fibroblast growth factor 2 (FGF-2), and
   (iv) at least one selected from the group consisting of a Wnt agonist, a bone morphogenetic protein (BMP) inhibitor, a transforming growth factor-$\beta$ (TGF-$\beta$) inhibitor, and an epidermal growth factor (EGF).

2. The production method according to claim 1,
   wherein the suspension culture is carried out while stirring the medium.

3. The production method according to claim 1 or 2,
   wherein the biological tissue is a normal tissue.

4. The production method according to any one of claims 1 to 3,

wherein the Wnt agonist is at least one selected from the group consisting of a Wnt protein, an R-spondin, and a GSK-3β inhibitor.

5. The production method according to any one of claims 1 to 4,
   wherein the Wnt agonist is a Wnt protein and an R-spondin.

6. The production method according to claim 5,
   wherein the Wnt protein is a complex with afamin.

7. A screening method for an anticancer drug, comprising:

   a step of carrying out suspension culture of a normal tissue-derived organoid and a tumor tissue-derived organoid in a state of being dispersed in a medium in a presence of a test substance; and
   a step of evaluating an effect of the test substance on the normal tissue-derived organoid and the tumor tissue-derived organoid,
   wherein the test substance that has few side effects on the normal tissue-derived organoid and suppresses proliferation of the tumor tissue-derived organoid is shown to be an anticancer drug that is effective against the tumor tissue, and
   the medium is substantially free of a p38 inhibitor and contains the following components (i) to (iv),

   (i) 0.1% to 10% by volume of an extracellular matrix,
   (ii) IGF-1,
   (iii) FGF-2, and
   (iv) at least one selected from the group consisting of a Wnt agonist, a BMP inhibitor, a TGF-β inhibitor, and an EGF.

8. The screening method according to claim 7,
   wherein the normal tissue-derived organoid is an organoid produced by the production method according to claim 3.

FIG. 1

EP 4 491 711 A1

# FIG. 2

Legend:
- ☐ EMBEDDED IN MATRIGEL
- ▨ SUSPENSION CULTURE
- ▨ SUSPENSION CULTURE WITH STIRRING

Y-axis: DOUBLING TIME (DAYS)

X-axis categories: CRC17b, CRC25a, CRC32, NLCRC32

FIG. 3

FIG. 4

# FIG. 5

FIG. 6

# FIG. 7

# FIG. 8

FIG. 9

# FIG. 10

FIG. 11

# FIG. 12

# FIG. 13

(+)-JQ1 [μM]

**EP 4 491 711 A1**

## INTERNATIONAL SEARCH REPORT

International application No.

**PCT/JP2023/008765**

### A. CLASSIFICATION OF SUBJECT MATTER

*C12N 5/071*(2010.01)i
FI: C12N5/071

According to International Patent Classification (IPC) or to both national classification and IPC

### B. FIELDS SEARCHED

Minimum documentation searched (classification system followed by classification symbols)

C12N5/071

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2020/235206 A1 (KEIO UNIVERSITY) 26 November 2020 (2020-11-26) example 1, claims, paragraphs [0075]-[0080] | 1-8 |
| Y | WO 2017/199811 A1 (KEIO UNIVERSITY) 23 November 2017 (2017-11-23) claims, examples paragraphs [0010]-[0095] | 1-8 |
| Y | JP 2021-519585 A (THE UNIVERSITY OF NORTH CAROLINA AT CHAPEL HILL) 12 August 2021 (2021-08-12) claims, examples | 1-8 |
| Y | WO 2021/113924 A1 (THE WALTER AND ELIZA HALL INSTITUTE OF MEDICAL RESEARCH) 17 June 2021 (2021-06-17) claims, examples | 1-8 |
| Y | CAPELING, M. M. et al. Suspension culture promotes serosal mesothelial development in human intestinal organoids. Cell Rep. 15 February 2022, vol. 38, no. 7, 110379, pp. 1-12, e1-e8, DOI:10.1016/j.celrep.2022.110379 abstract, fig. 1 | 1-8 |

☑ Further documents are listed in the continuation of Box C.     ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **16 May 2023** | **30 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| International application No. |
|---|
| **PCT/JP2023/008765** |

### C.     DOCUMENTS CONSIDERED TO BE RELEVANT

| Category* | Citation of document, with indication, where appropriate, of the relevant passages | Relevant to claim No. |
|---|---|---|
| A | JP 2014-516562 A (KONINKLIJKE NEDERLANDSE AKADEMIE VAN WETENSCHAPPEN) 17 July 2014 (2014-07-17)<br>      entire text | 1-8 |
| P, X | TOSHIMITSU, K. et al. Organoid screening reveals epigenetic vulnerabilities in human colorectal cancer. Nat Chem Biol. 10 March 2022, vol. 18, no. 6, pp. 605-614, DOI:10.1038/s41589-022-00984-x<br>      entire text | 1-8 |

Form PCT/ISA/210 (second sheet) (January 2015)

INTERNATIONAL SEARCH REPORT
Information on patent family members

International application No.

PCT/JP2023/008765

| Patent document cited in search report | | | Publication date (day/month/year) | Patent family member(s) | | | Publication date (day/month/year) |
|---|---|---|---|---|---|---|---|
| WO | 2020/235206 | A1 | 26 November 2020 | US 2022/0220427 A1<br>example 1, claims, paragraphs<br>[0117]-[0123]<br>EP 3974514 A1<br>CN 113840903 A | | | |
| WO | 2017/199811 | A1 | 23 November 2017 | US 2019/0390171 A1<br>claims, examples paragraphs<br>[0042]-[0136]<br>EP 3460042 A1<br>CN 109415680 A | | | |
| JP | 2021-519585 | A | 12 August 2021 | US 2019/0300849 A1<br>claims, examples<br>WO 2019/191402 A1<br>EP 3781181 A1<br>KR 10-2020-0140836 A<br>CN 112272698 A | | | |
| WO | 2021/113924 | A1 | 17 June 2021 | EP 4073232 A1<br>claims, examples | | | |
| JP | 2014-516562 | A | 17 July 2014 | US 2012/0196312 A1<br>whole document<br>WO 2012/168930 A2<br>EP 2412800 A1<br>CN 103237888 A<br>KR 10-2013-0138729 A | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63317552 B

**Non-patent literature cited in the description**

- **SATO T et al.** Single Lgr5 stem cells build crypt-villus structures in vitro without a mesenchymal niche. *Nature*, 2009, vol. 459, 262-266
- **SATO T et al.** Long-term Expansion of Epithelial Organoids From Human Colon, Adenoma, Adenocarcinoma, and Barrett's Epithelium. *Gastroenterology*, 2011, vol. 141, 1762-1772
- **FUJII M et al.** A Colorectal Tumor Organoid Library Demonstrates Progressive Loss of Niche Factor Requirements during Tumorigenesis. *Cell Stem Cell*, 2016, vol. 18, 827-838
- **SEINO T et al.** Human Pancreatic Tumor Organoids Reveal Loss of Stem Cell Niche Factor Dependence during Disease Progression. *Cell Stem Cell*, 2018, vol. 22, 454-467
- **NANKI K et al.** Divergent Routes toward Wnt and R-spondin Niche Independency during Human Gastric Carcinogenesis. *Cell*, 2018, vol. 174, 856-869

- *CHEMICAL ABSTRACTS*, 152121-30-7
- *CHEMICAL ABSTRACTS*, 152121-47-6
- *CHEMICAL ABSTRACTS*, 745833-23-2
- *CHEMICAL ABSTRACTS*, 209410-46-8
- *CHEMICAL ABSTRACTS*, 152121-53-4
- *CHEMICAL ABSTRACTS*, 1449811-01-2
- *CHEMICAL ABSTRACTS*, 285983-48-4
- *CHEMICAL ABSTRACTS*, 252917-06-9
- *CHEMICAL ABSTRACTS*, 142273-20-9
- *CHEMICAL ABSTRACTS*, 667463-62-9
- *CHEMICAL ABSTRACTS*, 909910-43-6
- *CHEMICAL ABSTRACTS*, 301836-41-9
- *CHEMICAL ABSTRACTS*, 694433-59-5
- *CHEMICAL ABSTRACTS*, 356559-20-1
- *CHEMICAL ABSTRACTS*, 396129-53-6
- *CHEMICAL ABSTRACTS*, 627536-09-8
- *CHEMICAL ABSTRACTS*, 446859-33-2
- *CHEMICAL ABSTRACTS*, 1268524-70-4